# EUROPÄISCHE PATENTANMELDUNG

(11) **EP 2 453 471 A2**
(43) Veröffentlichungstag der Anmeldung: **16.05.2012**
(21) Anmeldenummer: 11185353.7
(22) Anmeldetag: 17.10.2011
(51) Int. Cl.: H01L 21/48, H01L 23/498

(54) **Verfahren zur galvanischen Abscheidung einer Elektrode auf einem dielektrischen Substrat**

(30) Priorität: 15.11.2010 US 413493 P
(71) Anmelder: Dyconex AG, 8303 Bassersdorf (CH)
(72) Erfinder: Hauer, Marc, 8050 Zürich (CH)
(74) Vertreter: Lindner-Vogt, Karin L.

(57) **Zusammenfassung**

Die Erfindung betrifft ein Verfahren zur galvanischen Abscheidung einer Elektrode aus einem metallischen Elektrodenmaterial (40) auf einem dielektrischen Substrat (10), wobei folgende Schritte ausgeführt werden:
- Abscheiden einer elektrisch leitfähigen Polymerschicht (20);
- Maskieren der elektrisch leitfähigen Polymerschicht (20) mit einer Maske;
- galvanische Abscheidung des metallischen Elektrodenmaterials (40) auf der elektrisch leitfähigen Polymerschicht (20);
- Entfernen der Maske;
- Entfernen oder Deaktivieren der überschüssigen leitfähigen Polymerschicht (20).

## Beschreibung

Die Erfindung betrifft ein Verfahren zur galvanischen Abscheidung einer Elektrode auf einem dielektrischen Substrat.

Es ist sind verschiedene Methoden bekannt, metallische Elektroden auf einem Substrat abzuscheiden.

Bei einer ersten Methode wird zur Vermeidung einer Diffusion zwischen einem Elektrodenmetall und dem Substrat auf ein Basismetall, beispielsweise Cu, zuerst eine Sperrschicht, beispielsweise Ni, und dann das Elektrodenmetall, beispielsweise Au, aufplattiert. Die Elektrode besteht daher nicht nur aus dem Elektrodenmetall. Da eine Sperrschicht nicht perfekt ist, kann es zu einer Legierungsbildung und damit zu einer Kontamination des Elektrodenmetalls kommen. Auch können, bei Vorliegen von Inhomogenitäten in der Sperrschicht, dort Korrosionsprobleme auftreten.

Bei einer weiteren Methode erfolgt eine selektive Abscheidung des Elektrodenmetalls, beispielsweise Au, auf eine metallische Folie, beispielsweise eine Cu-Folie, die auf einem Träger angeordnet ist. Nach dem Abscheiden des Elektrodenmetalls wird die Folie vom Träger getrennt und mit der Elektrodenmetallseite in ein Basismaterial hineinlaminiert. Anschließend wird die Folie in einem Ätzschritt entfernt und nur das Elektrodenmetall bleibt auf dem Basismaterial zurück. Bei gut miteinander legierenden Metallen wie Cu und Au kann bei der Abscheidung und den folgenden Prozessen eine Interdiffusion dieser Metalle stattfinden. Die so erzeugten Verunreinigungen können beim Einsatz des Elektrodenmetalls für in einen tierischen oder menschlichen Körper implantierte Elektroden zu Problemen führen.

Weiterhin ist bekannt, eine Trägerfolie mit dem Elektrodenmetall zu besputtern, einen Fotoresist aufzubringen, zu strukturieren und mit einer galvanischen Abscheidung das Elektrodenmetall aufzuplattieren. Der Fotoresist wird entfernt und mit einem leichten Ätzschritt die aufgesputterte Schicht weggeätzt. Um eine gute Haftung des Elektrodenmetalls auf dem Substrat zu erreichen, wird meist eine weitere Metallschicht als Haftvermittler, beispielsweise Cr, benötigt. Zusätzlich zu den aufwändigen und zahlreichen Prozessschritten kann eine solche Haftvermittlerschicht die Biokompatibilität des Elektrodenmetalls ungünstig beeinflussen.

Der Erfindung liegt die Aufgabe zugrunde, ein Verfahren zur Abscheidung einer Elektrode auf einem dielektrischen Substrat zu schaffen, bei dem eine Fremdmaterialdiffusion in die Elektrode zumindest vermindert ist.

Die Aufgabe wird erfindungsgemäß durch die Merkmale des Anspruchs 1 gelöst. Günstige Ausgestaltungen und Vorteile der Erfindung ergeben sich aus den weiteren Ansprüchen und der Beschreibung.

Es wird ein Verfahren zur galvanischen Abscheidung einer Elektrode aus einem metallischen Elektrodenmaterial auf einem nichtleitenden dielektrischen Substrat vorgeschlagen, wobei folgende Schritte ausgeführt werden:
- Abscheiden einer elektrisch leitfähigen Polymerschicht auf dem Substrat;
- Maskieren der elektrisch leitfähigen Polymerschicht mit einer Maske;
- galvanische Abscheidung des metallischen Elektrodenmaterials auf der elektrisch leitfähigen Polymerschicht;
- Entfernen der Maske;
- Entfernen oder Deaktivieren der überschüssigen leitfähigen Polymerschicht.

Das Deaktivieren (= Minimierung der Leitfähigkeit) des überschüssigen leitfähigen Polymerschicht kann beispielsweise durch Reduktion oder Oxidation des Leitpolymers erfolgen. Alternativ kann das Leitpolymer, beispielsweise durch ein selektives chemisches Ätzen oder Plasmaätzen auch entfernt werden.

Vorteilhaft kann eine Kontamination der Elektrode mit einem anderen Metall verhindert werden, da das Elektrodenmaterial additiv auf dem Substrat aufgebracht wird und nicht über einen (metallischen) Zwischenträger auf dem Substrat deponiert wird. Es können Elektroden aus hochreinen Metallen auf dielektrischen Substraten, insbesondere auf Polymeren, erzeugt werden. So gelingt beispielsweise die Abscheidung reinen Goldes auf einem dielektrischen, d.h. nichtleitenden, organischen Substrat (z.B. Polyimid, LCP) ohne dass dieses durch andere Metalle verunreinigt wird. Nach erfolgter Abscheidung bleibt als Elektrode nur das Gold auf der Oberfläche des Substrats zurück. Damit können Elektroden geschaffen werden für Bauelemente, die zur Implantation in einen menschlichen oder tierischen Körper vorgesehen sind, welche biokompatible Eigenschaften aufweisen und bei denen keine Störung der Funktion oder des Implantationsbereichs durch unerwünschte metallische Komponenten in den Elektroden zu erwarten sind.

Es besteht zu keinem Zeitpunkt das Risiko einer unkontrollierten Legierung des metallischen Elektrodenmaterials mit einem anderen Metall. Das metallische Elektrodenmaterial wird nur additiv auf dem Substrat aufgebaut. Der notwendige Einsatz an Chemie und Material kann somit vermindert werden.

Gemäß einer vorteilhaften Weiterbildung kann ein Stromfluss bei der galvanischen Abscheidung überwiegend über die elektrisch leitfähige Polymerschicht erfolgen. Das Substrat wird bei der galvanischen Abscheidung nur in leitfähigen Bereichen beschichtet, so dass eine Abscheidung des metallischen Elektrodenmaterials nur auf freiliegenden, d.h. resistfreien Bereichen, der elektrisch leitfähigen Polymerschicht erfolgen kann. Mit galvanischer Abscheidung kann ein Metall grundsätzlich in sehr hoher Reinheit abgeschieden werden. Es kann eine hochreine Elektrode auf einem dielektrischen Substrat abgeschieden werden. Durch die elektrisch leitfähige Polymerschicht kann ein Einsatz von metallischen Zuleitungen auf dem Substrat vermieden werden, so dass keine Fremdmetalle in die Nähe des metallischen Elektrodenmaterials gebracht werden müssen, um den elektrischen Stromkreis bei der galvanischen Abscheidung zu schließen.

Weiterhin wird alternativ ein Verfahren zur galvanischen Abscheidung einer Elektrode aus einem metallischen Elektrodenmaterial auf einem dielektrischen Substrat vorgeschlagen, wobei folgende Schritte ausgeführt werden:
- Auftragen einer metallischen Leitschicht auf das Substrat;
- Strukturieren der metallischen Leitschicht zur Erzeugung von freiliegenden Bereichen auf dem Substrat;
- Abscheiden einer elektrisch leitfähigen Polymerschicht zumindest in den freiliegenden Bereichen;
- Maskieren der elektrisch leitfähigen Polymerschicht in den freiliegenden Bereichen und/oder Maskierung der übrigbleibenden Metallschicht mit einer Maske;
- galvanische Abscheidung des metallischen Elektrodenmaterials auf der elektrisch leitfähigen Polymerschicht;
- Entfernen der Maske;
- Entfernen oder Deaktivieren der überschüssigen leitfähigen Polymerschicht.

Beim Abscheiden der elektrisch leitfähigen Polymerschicht zumindest in den freiliegenden Bereichen kann je nach Abscheidungsprozess des leitfähigen Polymers die Abscheidung selektiv auf dem freiliegenden Polymer erfolgen oder auf der gesamten Fläche, einschließlich auf dem metallischen Elektrodenmaterial. Günstige Abscheideprozesse sind z.B. aus wässriger Lösung selektiv nur auf dem Polymer mit Enthone Envision HDI (ehemals DMS-E Prozess) der Firma Enthone, Inc., in West Haven, Connecticut, USA, aus wässriger Lösung selektiv nur auf dem Polymer mit Atotech Seleo CP Plus der Firma Atotech Deutschland GmbH in Berlin, oder eine nicht selektive Abscheidung aus der Gasphase mittels Plasmabeschichtung.

Das Deaktivieren (= Minimierung der Leitfähigkeit) des überschüssigen leitfähigen Polymerschicht kann beispielsweise durch Reduktion oder Oxidation des Leitpolymers erfolgen. Alternativ kann das Leitpolymer, beispielsweise durch ein selektives chemisches Ätzen, oder ein Plasmaätzen, auch entfernt werden.

Günstige Materialien für elektrisch leiterfähige Polymere sind beispielsweise Polyanilin, Polypyrrol, Polythiophen.

Die Dicke der Polymerschicht sollte so dünn wie möglich sein, da diese leichter zu entfernen ist, jedoch so dick, dass eine ausreichende elektrische Leitfähigkeit für die Metallabscheidung gegeben ist. Eine sinnvolle Dicke kann beispielsweise unter höchstens 10µm liegen. Genaue Grenzen können abhängig vom aktuellen System variieren und leicht durch Tests ermittelt werden. Eine Dicke der Edelmetallschicht ist definiert durch die notwendige Stabilität des Leiters und die Stromstärke bzw. des Widerstandes der für die Anwendung akzeptabel ist. Der Vorteil einer solchen Abscheidung gegenüber einer direkten Edelmetall Abscheidung aus der Gasphase ist, dass höhere Edelmetalldicken sinnvoll abgeschieden werden können. Die maximale Leiterdicke ist praktisch nur durch die Resist-Dicke beschränkt. Günstige Leiterdicken können beispielsweise in einem Bereich von 0.5 µm bis 100 µm liegen.

Vorteilhaft kann bei der alternativen Variante eine Kontamination der Elektrode mit einem anderen Metall verhindert werden, da das Elektrodenmaterial additiv auf dem Substrat aufgebracht wird und nicht über einen (metallischen) Zwischenträger auf dem Substrat deponiert wird. Es können Elektroden aus hochreinen Metallen auf dielektrischen Substraten, insbesondere Polymeren, erzeugt werden. So gelingt beispielsweise die Abscheidung von reinem Gold auf einem dielektrischen, d.h. nichtleitenden, organischen Substrat (z.B. Polyimid, LCP) ohne dass dieses durch andere Metalle verunreinigt wird. Nach erfolgter Abscheidung bleibt als Elektrode nur das Gold auf der Oberfläche des Substrats zurück. Damit können Elektroden geschaffen werden für Bauelemente, die zur Implantation in einen menschlichen oder tierischen Körper vorgesehen sind, welche biokompatible Eigenschaften aufweisen und bei denen keine Störung durch unerwünschte metallische Komponenten in den Elektroden zu erwarten sind.

Es besteht zu keinem Zeitpunkt das Risiko einer unkontrollierten Legierung des metallischen Elektrodenmaterials mit einem anderen Metall. Das metallische Elektrodenmaterial wird nur additiv auf dem Substrat aufgebaut. Der notwendige Einsatz an Chemie und Material kann somit vermindert werden.

Gemäß einer vorteilhaften Weiterbildung der alternativen Variante kann die metallische Leitschicht bei der galvanischen Abscheidung durch einen Teil der Maske von dem Bereich getrennt werden, in dem das metallischen Elektrodenmaterial abgeschieden werden soll, wobei die metallische Leitschicht den Bereich eng umschließt. Damit kann ein Teil des Stromflusses während der galvanischen Abscheidung über die relativ niederohmige metallische Leitschicht erfolgen.

Gemäß einer vorteilhaften Weiterbildung bei der alternativen Variante kann ein Stromfluss bei der galvanischen Abscheidung überwiegend über die Leitschicht erfolgen. Dies erfolgt vorteilhaft bis in die Nähe der galvanisch abzuscheidenden Elektrode. In dem Bereich zwischen Leitschicht und abzuscheidender Elektrode kann die elektrisch leitfähige Polymerschicht den Stromfluss tragen. Die metallische Leitschicht kommt nicht in Kontakt mit dem metallischen Elektrodenmaterial. Verglichen mit der elektrisch leitfähigen Polymerschicht ist die metallische Leitschicht niederohmiger, so dass eine bessere Stromverteilung und damit eine bessere Schichtdickenverteilung des Elektrodenmetalls erreicht wird.

Das Substrat wird bei der galvanischen Abscheidung nur in leitfähigen Bereichen beschichtet, so dass eine Abscheidung des metallischen Elektrodenmaterials nur auf der elektrisch leitfähigen Polymerschicht erfolgen kann, da die Leitschicht während der galvanischen Abscheidung zweckmäßigerweise mit Fotoresist abgedeckt ist. Durch die elektrisch leitfähige Polymerschicht im Bereich, in dem das metallische elektrodenmaterial abgeschieden werden soll, kann ein Einsatz von metallischen Zuleitungen in diesem Bereich vermieden werden, so dass keine Fremdmetalle mit dem metallischen Elektrodenmaterials in Berührung kommen, um den elektrischen Stromkreis bei der galvanischen Abscheidung zu schließen.

Gemäß einer vorteilhaften Weiterbildung bei der alternativen Variante kann die Leitschicht vor der galvanischen Abscheidung des metallischen Elektrodenmaterials mit einer Diffusionssperrschicht beschichtet werden. Damit ist die Gefahr einer Kontamination des metallischen Elektrodenmaterials mit dem Material der metallischen Leitschicht weiter verringert. Gemäß einer vorteilhaften Weiterbildung bei der alternativen Variante kann als Diffusionssperrschicht Nickel und/oder Palladium eingesetzt werden. Diese Materialien zeichnen sich unter anderem durch eine wirksame Hemmung von Diffusionsvorgängen aus.

Für beide Varianten des vorgeschlagenen Verfahrens kann günstigerweise die Maskierung der elektrisch leitfähigen Polymerschicht durch eine Fotoresist-Maske erfolgen. Ein Fotoresist lässt sich leicht aufbringen, strukturieren und entfernen. Die Oberfläche der abgeschiedenen Elektrode kommt bei der galvanischen Abscheidung nicht mit dem Fotoresist in Berührung, und beim Entfernen des Fotoresists nur kurzzeitig im Lösungsmittel oder praktisch gar nicht bei einer physikalischen Ätzmethode oder Oxidationsmethode.

Für beide Varianten des vorgeschlagenen Verfahrens kann günstigerweise die elektrisch leitfähige Polymerschicht außerhalb des abgeschiedenen metallischen Elektrodenmaterials entfernt werden oder die elektrische Leitfähigkeit der elektrisch leitfähigen Polymerschicht außerhalb des abgeschiedenen metallischen Elektrodenmaterials verringert werden.

Für beide Varianten des vorgeschlagenen Verfahrens kann als metallisches Elektrodenmaterial ein Edelmetall oder eine Legierung von Edelmetallen (z.B. Platin/Iridium) eingesetzt werden. Günstig ist beispielsweise Gold oder Platin als metallisches Elektrodenmaterial. Als organisches Substrat eignet sich ein Polymer, welches für in den menschlichen oder tierischen Körper implantierbare Bauelemente mit Elektroden gut geeignet ist, insbesondere PI (PI = Polyimid) oder LCP (LCP = Liquid Crystal Polymer). Die Verfahren können insgesamt bei niedrigen Temperaturen, z.B. Raumtemperatur, betrieben werden, womit das organische Substrat vor der Einwirkung überhöhter Temperaturen geschützt ist.

Die Erfindung ist nachfolgend beispielhaft, anhand von in Zeichnungen dargestellten Ausführungsbeispielen, näher erläutert. Es zeigen in schematischer Darstellung:
- Fig. 1A-1F: verschiedene Verfahrensschritte bei einer galvanischen Beschichtung eines dielektrischen Substrats mit einem metallischen Elektrodenmaterial;
- Fig. 2A-2H: verschiedene Verfahrensschritte bei einer galvanischen Beschichtung eines dielektrischen Substrats mit einem metallischen Elektrodenmaterial unter Verwendung einer metallischen Leitschicht;
- Fig. 3A-3H: verschiedene Verfahrensschritte bei einer alternativen galvanischen Beschichtung eines dielektrischen Substrats mit einem metallischen Elektrodenmaterial unter Verwendung einer an selektiven Stellen mit einer Sperrschicht in Kontakt stehenden metallischen Leitschicht.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

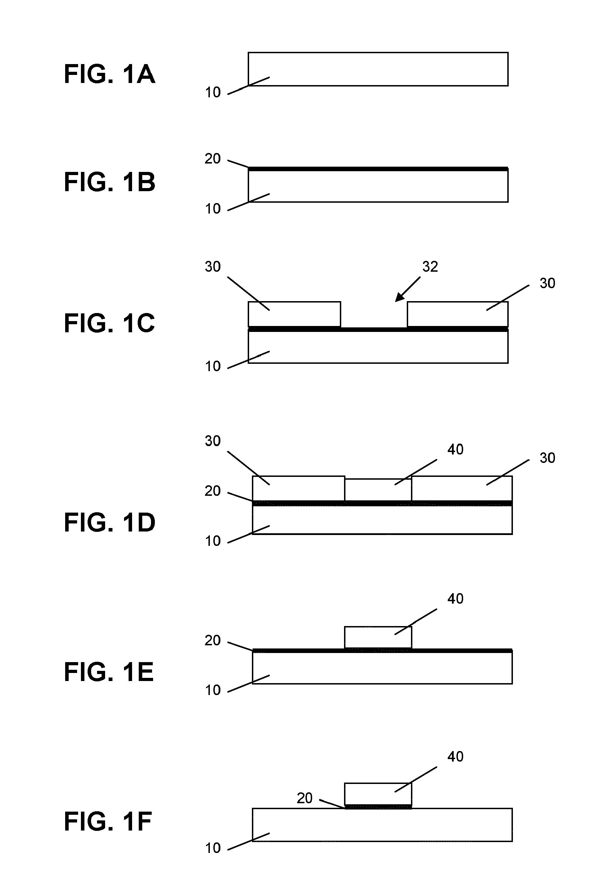

Die Fig. 1A-1E zeigen anhand von Schnittdarstellungen verschiedener Verfahrensschritte bei einer Beschichtung eines dielektrischen, insbesondere organischen Substrats 10 mit einem reinen metallischen Elektrodenmaterial 40, insbesondere einem Edelmetall, z.B. Gold. Das metallische Elektrodenmaterial 40 kann dabei z.B. in Form einer Leiterbahn auf dem Substrat 10 abgeschieden werden. Geeignete Schichtdicken liegen beispielsweise zwischen 1 µm und 20 µm, es können jedoch auch größere Schichtdicken, z.B. bis 100µm eingesetzt werden. Selbstverständlich können auch mehrere Elektroden aus metallischen Elektrodenmaterial 40 abgeschieden werden.

Ein dielektrisches organisches Substrat 10, insbesondere aus einem biokompatiblen Polymer wie PI oder LCP, wird bereitgestellt (Fig. 1A) und beispielsweise ganzflächig eine elektrisch leitfähige Polymerschicht 20 abgeschieden (Fig. 1B).

Die elektrisch leitfähige Polymerschicht 20 wird mit einer Maske versehen, indem ein Fotoresist aufgetragen und strukturiert wird, wobei Bereiche 30 der elektrisch leitfähige Polymerschicht 20 abgedeckt und ein Bereich 32 der elektrisch leitfähigen Polymerschicht 20 freigelegt sind (Fig. 1C).

Auf dem freigelegten Bereich 32 der elektrisch leitfähigen Polymerschicht 20 wird das metallische Elektrodenmaterial 40 galvanisch abgeschieden, wobei die elektrisch leitfähige Polymerschicht 20 als Stromzuleitung dient (Fig. 1D). Anschließend wird der Fotoresist entfernt (Fig. 1E), so dass das reine Elektrodenmaterial 40 auf dem Substrat 10 zurückbleibt. Im letzten Schritt (Fig. 1F) wird die freiliegende elektrisch leitfähige Polymerschicht 20 um das metallische Elektrodenmaterial 40 herum z.B. durch chemisches und/oder Plasmaätzen entfernt. Alternativ kann stattdessen die elektrische Leitfähigkeit der freiliegenden elektrisch leitfähigen Polymerschicht 20 reduziert werden. Dies kann z.B. durch eine chemische Oxidation oder Reduktion erfolgen.

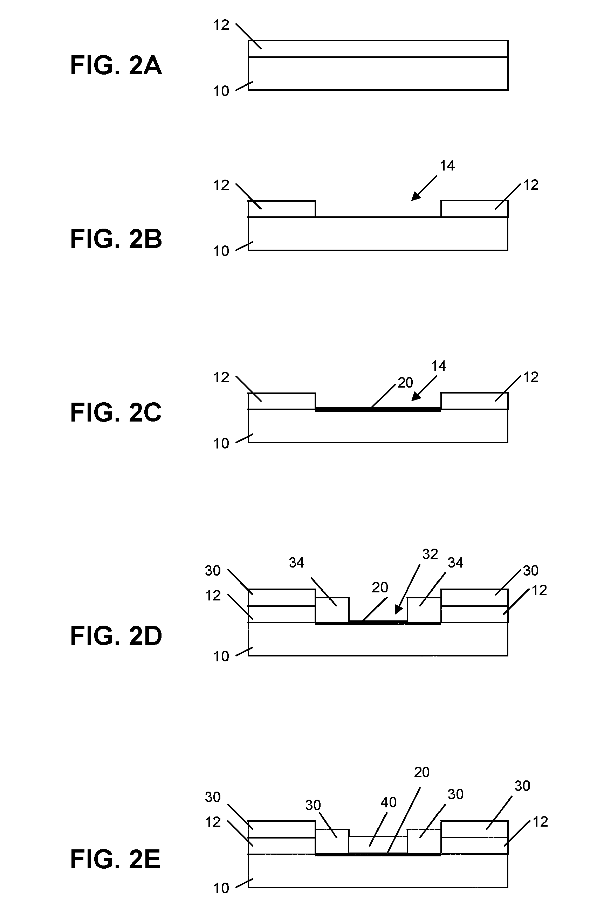

Fig. 2A-2H sind anhand von Schnittdarstellungen verschiedener Verfahrensschritte bei einer Beschichtung eines dielektrischen Substrats 10 mit einem metallischen Elektrodenmaterial 40, insbesondere einem Edelmetall, z.B. Gold, unter Verwendung einer metallischen Leitschicht 12, beispielsweise aus Kupfer, erläutert. Das metallische Elektrodenmaterial 40 kann dabei z.B. in Form einer Leiterbahn auf dem Substrat 10 abgeschieden werden. Selbstverständlich können auch mehrere Elektroden aus metallischen Elektrodenmaterial 40 abgeschieden werden

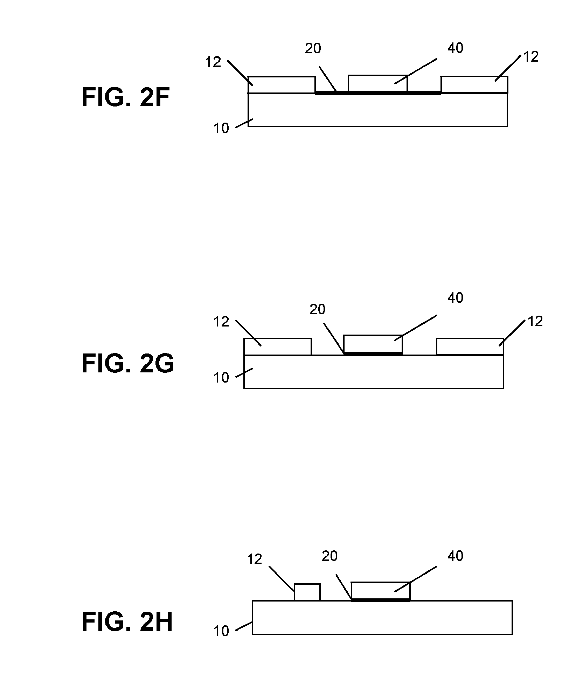

Auf einem dielektrischen Substrat 10, insbesondere aus einem biokompatiblen Polymer wie PI oder LCP oder dergleichen, wird eine metallische Leitschicht 12 aufgebracht, insbesondere auflaminiert oder durch ein Beschichtungsverfahren abgeschieden (Fig. 2A). Die Leitschicht 12 wird zur Erzeugung von einem freiliegenden Bereich 14 auf dem Substrat 10 strukturiert (Fig. 2B). In den freiliegenden Bereich 14 des Substrats 10 wird eine elektrisch leitfähige Polymerschicht 20 abgeschieden (Fig. 2C). Eine Abscheidung auf der Leiterschicht stört nicht, wenn nach dem Abscheiden des metallischen Elektrodenmaterials 40 die leitfähige Polymerschicht 20 überall entfernt wird, wie in Fig. 2F und 2G gezeigt, bzw. wenn die leitfähige Polymerschicht 20 den Ätzschritt in Fig. 2G und Fig. 2H nicht stört.

Die Leitschicht 12 sowie die in dem freiliegenden Bereich 14 angeordnete elektrisch leitfähige Polymerschicht 20 werden mit einer Maske aus einem Fotoresist maskiert, wobei abgedeckte Bereiche 30 der Leitschicht 12 und ein Bereich 34 der elektrisch leitfähigen Polymerschicht 20 abgedeckt werden und ein Bereich 32 der elektrisch leitfähigen Polymerschicht 20 freigelegt wird (Fig. 2D). Die Leitschicht 12 wird eng an den zu beschichtenden, freiliegenden Bereich 32 der elektrisch leitfähigen Polymerschicht 20 herangeführt, jedoch durch die Bereiche 34 des Fotoresists von dem freiliegenden Bereich 32 getrennt.

Es erfolgt eine galvanische Abscheidung des metallischen Elektrodenmaterials 40 auf die freiliegenden Bereiche 32 der elektrisch leitfähigen Polymerschicht 20 (Fig. 2E). Der Stromfluss bei der galvanischen Abscheidung erfolgt wesentlich über die (abgedeckte) Leitschicht 12 und nur in unmittelbarer Nähe zum zu beschichtenden Bereich 32 über die elektrisch leitfähige Polymerschicht 20. Trotz der verglichen mit der Leitschicht 12 relativ geringen elektrischen Leitfähigkeit der elektrisch leitfähigen Polymerschicht 20 kann eine homogene galvanische Abscheidung auf der freiliegenden elektrisch leitfähigen Polymerschicht 20 erfolgen. Durch die Abdeckung durch die Bereiche 34 gerät das Metall der Leitschicht 12 nicht in direkten Kontakt mit dem abgeschiedenen metallischen Elektrodenmaterial 40, so dass eine Interdiffusion zwischen den beiden Metallen unterbunden oder zumindest minimiert ist.

Nach Entfernen des Fotoresists um das abgeschiedene metallische Elektrodenmaterial 40 (Fig. 2F) wird die überschüssige freiliegende elektrisch leitfähigen Polymerschicht 20 entfernt, etwa durch chemisches oder Plasmaätzen oder chemische Oxidation, oder zumindest dessen elektrische Leitfähigkeit stark reduziert (Fig. 2G). Die Leitschicht 12 kann dann selektiv entfernt, z.B. selektiv geätzt werden. Dazu kann ein Fotoresist mit einer Negativmaske zu der Struktur des Fotoresist in Fig. 2D aufgetragen werden.

Ein Teil der Leitschicht 12 kann dort zurückbleiben, wo auf der gleichen Ebene des Substrats 10 nur elektrische Ströme transportiert werden und eine Funktion als Elektrode also nicht notwendig ist. Dadurch kann die Menge an abgeschiedenem metallischem Elektrodenmaterial 40 minimiert werden. Ist eine elektrische Kontaktierung zwischen der Rest-Leitschicht 12 und der Elektrode aus metallischem Elektrodenmaterial 40 notwendig, so kann die Leitschicht 12 im Kontaktbereich lokal mit einer Diffusionssperrschicht, z.B. aus Nickel oder Palladium, beschichtet werden.

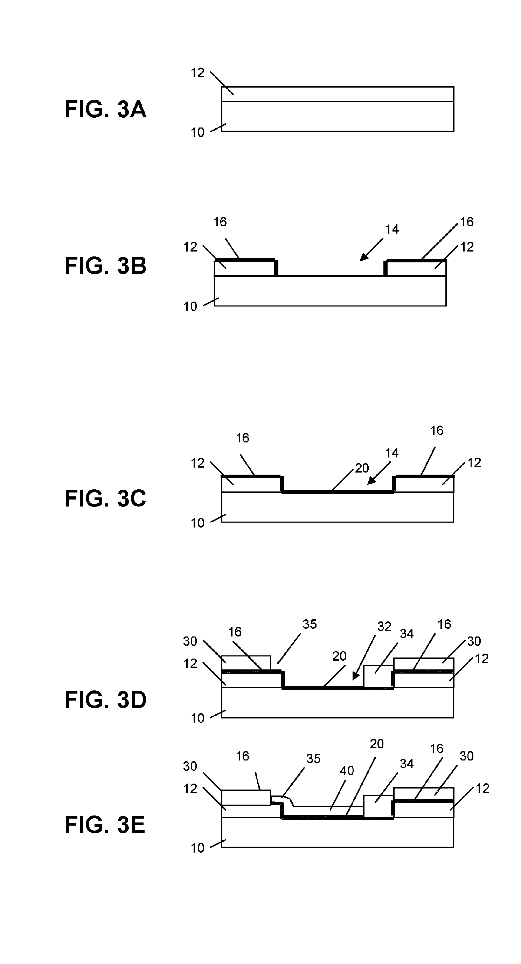

Um das metallische Elektrodenmaterial 40 elektrisch an die metallische Leitschicht 12 anzuschließen, kann dies über die in den Fig. 3A-3H beschriebenen Prozesskette erzeugt werden. Dabei wird durch das Aufbringen einer Diffusionssperrschicht 16 im Verbindungsbereich eine Diffusion in die Elektrode aus metallischem Elektrodenmaterial 40 unterbunden oder zumindest vermindert. Ist der Verbindungsbereich räumlich weit, z.B. einige Millimeter, vom Elektrodenbereich entfernt, so kann eine etwaig auftretende Restdiffusion vernachlässigt werden. Handelt es sich bei der Diffusionsbarriere 16 um ein Metall, das gleichzeitig mit der metallischen Leitschicht 12 geätzt werden kann (z.B. Cu und Ni), dann kann die Diffusionsbarriere 16 vollflächig aufgebracht werden.

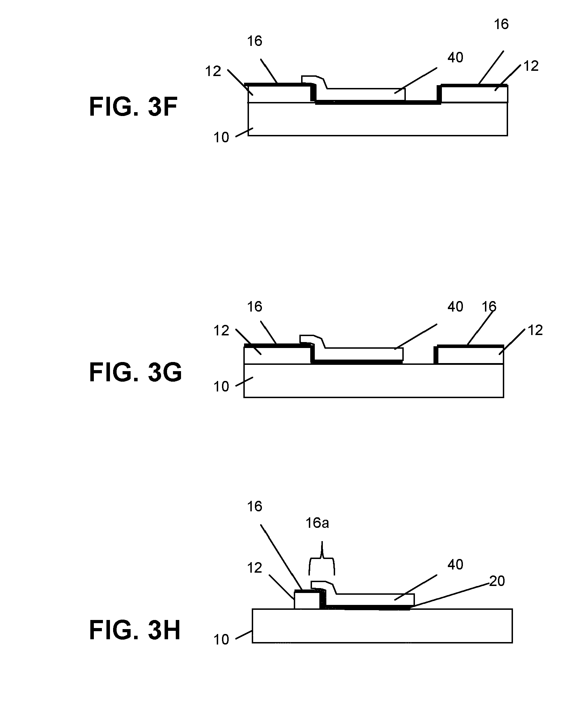

Auf einem dielektrischen Substrat 10, insbesondere aus einem biokompatiblen Polymer wie PI oder LCP oder dergleichen, wird eine metallische Leitschicht 12 aufgebracht, d.h. auflaminiert oder durch einen Abscheideprozess abgeschieden (Fig. 3A). Die Leitschicht 12 wird zur Erzeugung eines freiliegenden Bereichs 14 auf dem Substrat 10 strukturiert und eine Diffusionssperrschicht 16, z.B. aus Nickel oder Palladium, auf der Leitschicht 12 abgeschieden (Fig. 3B). Diese Variante ist günstig, wenn das metallische Elektrodenmaterial 40 elektrisch an die metallische Leitschicht 12 angeschlossen werden soll. In den freiliegenden Bereich 14 des Substrats 10 wird eine elektrisch leitfähige Polymerschicht 20 abgeschieden (Fig. 3C). Eine Abscheidung auf der metallischen Leitschicht 12 stört nicht, wenn nach dem Abscheiden des metallischen Elektrodenmaterials 40 die leitfähige Polymerschicht 20 überall entfernt wird, wie in Fig. 3F, 3G gezeigt, bzw. wenn die leitfähige Polymerschicht 20 den Ätzschritt in Fig. 3G, 3H nicht stört.

Die Leitschicht 12 sowie die in dem freiliegenden Bereich 14 angeordnete elektrisch leitfähige Polymerschicht 20 werden mit einer Maske aus einem Fotoresist maskiert, wobei abgedeckte Bereiche 30 der Leitschicht 12 und ein Bereich 34 der elektrisch leitfähigen Polymerschicht 20 abgedeckt werden und ein Bereich 32 der elektrisch leitfähigen Polymerschicht 20, sowie ein Bereich 35 der Leitschicht freigelegt wird (Fig. 3D). Da eine perfekte Registrierung nicht möglich ist, muss zusätzlich ein kleiner Bereich über der Leitschicht freigelegt werden.

Es erfolgt eine galvanische Abscheidung des metallischen Elektrodenmaterials 40 auf die freiliegenden Bereiche 34 der elektrisch leitfähigen Polymerschicht 20 (Fig. 3E) und Bereich 35 auf der freiliegenden Leitschicht. Der Stromfluss bei der galvanischen Abscheidung erfolgt wesentlich über die (abgedeckte) Leitschicht 12 und in unmittelbarer Nähe zum zu beschichtenden Bereich 32 über die elektrisch leitfähige Polymerschicht 20. Trotz der verglichen mit der Leitschicht 12 relativ geringen elektrischen Leitfähigkeit der elektrisch leitfähigen Polymerschicht 20 kann eine homogene galvanische Abscheidung auf der freiliegenden elektrisch leitfähigen Polymerschicht 20 erfolgen. Das galvanisch abgeschiedene Elektrodenmetall steht nur im selektiven Bereich 35 mit dem Leitmetall in Kontakt. Die Diffusion des Leitmetalls in das Elektrodenmetall ist dort bereits durch eine Diffusionssperre vom Elektrodenmaterial minimiert. Da der Kontakt zwischen beiden Metallen nur sehr lokal ist, kann der Kontaktbereich so gewählt werden dass dieser Räumlich sehr weit von aktiven Elektrodenoberfläche entfernt ist so dass das Risiko der Diffusion weiter minimiert werden kann.

Nach Entfernen des Fotoresists um das abgeschiedene metallische Elektrodenmaterial 40 (Fig. 3F) wird die überschüssige freiliegende elektrisch leitfähigen Polymerschicht 20 entfernt, etwa durch chemisches oder Plasmaätzen, oder zumindest dessen elektrische Leitfähigkeit stark reduziert (Fig. 3G). Die Leitschicht 12 und die Diffusionsschicht 16 kann dann selektiv entfernt, z.B. selektiv geätzt werden. Dazu kann ein Fotoresist mit einer Negativmaske zu der Struktur des Fotoresist in Fig. 3D aufgetragen werden.

Ein Teil der Leitschicht 12 kann dort zurückbleiben, wo auf der gleichen Ebene des Substrats 10 nur elektrische Ströme transportiert werden und eine Funktion als Elektrode also nicht notwendig ist (Fig. 3H). Dadurch kann die Menge an abgeschiedenem metallischem Elektrodenmaterial 40 minimiert werden. Ist eine elektrische Kontaktierung zwischen beiden der Rest-Leitschicht 12 und der Elektrode aus metallischem Elektrodenmaterial 40 notwendig, so schützt die Diffusionssperrschicht 16 die Leitschicht 12 lokal im Kontaktbereich 16a. Dieser Bereich ist räumlich hinreichend weit vom eigentlichen Elektrodenbereich entfernt. Wird z.B. Nickel als Diffusionssperrschicht 16 verwendet, so kann das Nickel mit dem Kupfer geätzt werden und stört diesen letzten Ätzschritt nicht. Sollte die Diffusionsschicht die weiteren Prozesse stören, so können diese durch ein selektives Ätzen der Leitschicht entfernt werden. Die Diffusionsschicht bleibt dabei im Interface Bereich (16a) weiter bestehen.

Diese Ausgestaltung ist dann günstig, wenn das derart beschichtete Substrat 10 nicht nur als biokompatible Elektrode, sondern auch als Träger für Komponenten oder als Kabel außerhalb des Körpers verwendet werden soll. Es ergibt sich ein Kostenvorteil, wenn nur die kritischen Bereiche in Edelmetall ausgeführt werden. Die unkritischen Bereiche können aus der metallischen Leitschicht 12, die sich ja bereits auf dem Substrat 10 befindet, hergestellt werden.

Mit der Erfindung gelingt ein strukturierter galvanischer Aufbau von Metallleitern (z.B. Au) auf einem dielektrischen Basismaterial (Substrat 10) mittels eines Leitpolymers
Das metallische Elektrodenmaterial wird nur additiv aufgebaut und der notwendige Einsatz an Chemie und Material ist somit minimiert.

## Patentansprüche

1. Verfahren zur galvanischen Abscheidung einer Elektrode aus einem metallischen Elektrodenmaterial (40) auf einem dielektrischen Substrat (10), wobei folgende Schritte ausgeführt werden:
- Abscheiden einer elektrisch leitfähigen Polymerschicht (20) auf dem Substrat (10);
- Maskieren der elektrisch leitfähigen Polymerschicht (20) mit einer Maske;
- galvanische Abscheidung des metallischen Elektrodenmaterials (40) auf der elektrisch leitfähigen Polymerschicht (20);
- Entfernen der Maske;
- Entfernen oder Deaktivieren der überschüssigen leitfähigen Polymerschicht (20).

2. Verfahren nach Anspruch 1, wobei ein Stromfluss bei der galvanischen Abscheidung überwiegend über die elektrisch leitfähige Polymerschicht (20) erfolgt.

3. Verfahren zur galvanischen Abscheidung einer Elektrode aus einem metallischen Elektrodenmaterial (40) auf einem dielektrischen Substrat (10), wobei folgende Schritte ausgeführt werden:
- Auftragen einer Leitschicht (12) auf das organische Substrat (10);
- Strukturieren der Leitschicht (12) zur Erzeugung von freiliegenden Bereichen (14) auf dem Substrat (10);
- Abscheiden einer elektrisch leitfähigen Polymerschicht (20) zumindest in den freiliegenden Bereichen (14);
- Maskieren der Leitschicht (12) und der elektrisch leitfähigen Polymerschicht (20) in den freiliegenden Bereichen (14) und/oder Maskierung der übrigbleibenden Metallschicht mit einer Maske;
- galvanische Abscheidung des metallischen Elektrodenmaterials (40) auf der elektrisch leitfähigen Polymerschicht (20);
- Entfernen der Maske;
- Entfernen oder Deaktivieren der überschüssigen leitfähigen Polymerschicht (20).

4. Verfahren nach Anspruch 3, wobei die Leitschicht (12) bei der galvanischen Abscheidung durch einen Teil (32) der Maske (30) von dem Bereich (32) getrennt wird, der zur Abscheidung des metallischen Elektrodenmaterials (40) vorgesehen ist, wobei die Leitschicht (12) den Bereich (32) eng umschließt.

5. Verfahren nach Anspruch 3 oder 4, wobei ein Stromfluss bei der galvanischen Abscheidung überwiegend über die Leitschicht (12) erfolgt.

6. Verfahren nach einem der Ansprüche 3 bis 5, wobei die Leitschicht (12) vor der galvanischen Abscheidung des metallischen Elektrodenmaterials (40) mit einer Diffusionssperrschicht (16) beschichtet wird.

7. Verfahren nach einem der Ansprüche 3 bis 6, wobei die Leitschicht (12) zumindest bereichsweise entfernt wird.

8. Verfahren nach einem der vorhergehenden Ansprüche, wobei die Maskierung der elektrisch leitfähigen Polymerschicht (20) durch eine Fotoresist-Maske erfolgt.

9. Verfahren nach einem der vorhergehenden Ansprüche, wobei die elektrisch leitfähige Polymerschicht (20) außerhalb des abgeschiedenen metallischen Elektrodenmaterials entfernt wird oder eine elektrische Leitfähigkeit der elektrisch leitfähigen Polymerschicht (20) außerhalb des abgeschiedenen metallischen Elektrodenmaterials (40) verringert wird.

10. Verfahren nach einem der vorhergehenden Ansprüche, wobei als metallisches Elektrodenmaterial (40) ein Edelmetall oder eine Legierung von Edelmetallen eingesetzt wird.
